(19) Europäisches Patentamt
European Patent Office
Office européen des brevets

(11) EP 4 384 163 B1

(12) **EUROPEAN PATENT SPECIFICATION**

(45) Date of publication and mention of the grant of the patent:
**25.03.2026 Bulletin 2026/13**

(21) Application number: **22762041.6**

(22) Date of filing: **05.08.2022**

(51) International Patent Classification (IPC):
***A61K 31/381*** (2006.01) ***A61P 1/16*** (2006.01)
***A61P 35/00*** (2006.01)

(52) Cooperative Patent Classification (CPC):
**A61K 31/381; A61P 1/16; A61P 35/00**

(86) International application number:
**PCT/EP2022/072165**

(87) International publication number:
**WO 2023/016950 (16.02.2023 Gazette 2023/07)**

(54) **SMALL MOLECULE TREATMENT OF FATTY LIVER DISEASE AND HCC WITH JNK-IN-5A**

KLEINMOLEKÜLIGE BEHANDLUNG VON FETTLEBERERKRANKUNG UND HCC MIT JNK-IN-5A

TRAITEMENT À PETITES MOLÉCULES D’UNE MALADIE DU FOIE GRAS NON ALCOOLIQUE (NAFLD) OU D’UN CARCINOME HÉPATOCELLULAIRE (HCC) AVEC JNK-IN-5A

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.08.2021 EP 21191191**

(43) Date of publication of application:
**19.06.2024 Bulletin 2024/25**

(73) Proprietor: **ScandiEdge Therapeutics AB**
**118 26 Stockholm (SE)**

(72) Inventors:
- **MARDINOGLU, Adil**
  **412 60 Göteborg (SE)**
- **BORÉN, Jan**
  **414 67 Göteborg (SE)**
- **UHLÉN, Mathias**
  **181 90 Lidingö (SE)**

(74) Representative: **Kransell & Wennborg KB**
**P.O. Box 27834**
**115 93 Stockholm (SE)**

(56) References cited:
**EP-A1- 3 444 010** **WO-A1-2014/114186**
**WO-A1-2018/195450**

- **CZAJA ET AL: "JNK regulation of hepatic manifestations of the metabolic syndrome", TRENDS IN ENDOCRINOLOGY AND METABOLISM, ELSEVIER SIENCE PUBLISHING , NEW YORK , NY, US, vol. 21, no. 12, 1 December 2010 (2010-12-01), pages 707 - 713, XP027510478, ISSN: 1043-2760, [retrieved on 20101001], DOI: 10.1016/J.TEM.2010.08.010**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 384 163 B1

## Description

## TECHNICAL FIELD

The present disclosure relates to the field of treatment of fatty liver disease and hepatocellular carcinoma (HCC).

## BACKGROUND

Non-alcoholic fatty liver disease (NAFLD) is defined as the accumulation of fat in the liver due to the imbalance in the uptake and secretion of fat as well as the increased *de novo* lipogenesis (DNL) or decreased oxidation of fat in the liver (Gluchowski et al., 2017). NAFLD can progress to non-alcoholic steatohepatitis (NASH). It has been reported that the prevalence of NAFLD is approximately 25% of the population, but it has no approved effective pharmacological treatment. Hence, there is an urgent need for the development of an effective treatment strategy.

Previously, the research groups including the present inventors have generated gene co-expression networks (CNs) for the liver and 45 other primary human tissues, and identified the pyruvate kinase L/R (PKLR) as a target, whose inhibition may selectively inhibit DNL in the liver (Lee et al., 2017). The PKLR gene encodes for the liver (PKL) and erythrocyte (PKR) isoforms of the enzyme and catalyses the production of pyruvate and ATP from phosphoenolpyruvate and ADP. PKL and PKR isoforms are specifically expressed in the liver and erythrocytes, respectively. An independent mouse population study has also verified the driving role of PKLR in the development of NAFLD (Chella Krishnan et al., 2018). Recently, *in vitro* experiments were performed by inhibiting and overexpressing PKLR in HepG2 cells, and it was found that the expression of PKLR was significantly positively correlated with the expression of FASN, *de novo* lipogenesis (DNL), triacylglycerol (TAG)levels and cell growth (Liu et al., 2019). WO2019034657 teaches a medical agent comprising a polynucleotide, which medical agent is capable of silencing the PKLR gene for treating a subject having non-alcoholic fatty liver disease (NAFLD) or hepatocellular carcinoma (HCC).

## SUMMARY

In the light of these findings presented above, it is suggested that PKLR can be targeted in the development of a fatty liver disease treatment with minimum side effects.

The present disclosure presents an investigational study of the driving role of PKLR in NAFLD progression using animal models and identifies small molecular drugs for inhibition of PKLR based on systems biology and drug repositioning analysis.

First, mice were fed with three different diets, including chow diet (CHOW), high sucrose diet (HSD) and high-fat diet (HFD), to investigate the modulation of the PKLR and other functionally associated protein-coding genes in response to different diets. Second, liver transcriptomic data was generated to reveal the molecular differences involved in the progression of NAFLD with different diets. The data was analysed using CNs and the appropriate diet for further animal experiments was selected. Third, the critical role of the PKLR in NAFLD development was investigated by generating a PKL knock out (KO) mouse model and feeding the animals with CHOW and HSD. Liver, muscle, white adipose tissue (WAT) and heart samples were collected from these animals and transcriptomic data was generated to reveal the underlying molecular mechanisms associated with the preventive role of PKL KO. Multi-tissue analysis was performed to identify the protective effect of PKL KO in NAFLD progression. Finally, a drug repositioning project was performed, which identified small molecule drugs candidates for modulation of the expression level of PKLR. The effects of two selected small molecules were tested *in vitro* and *in vivo* (rat study). One of the small molecules (JNK-IN-5A) was shown to be particularly suitable for treatment of fatty liver disease (such as NAFLD). Further, this molecule was also shown to inhibit the growth of HepG2 tumour cells, which extends the medical indication to hepatocellular carcinoma (HCC).

Accordingly, the present disclosure provides a compound of formula (I)

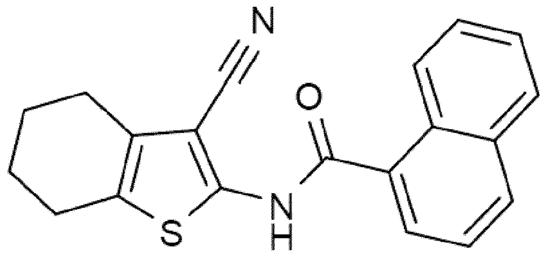

(I)

or a pharmaceutically acceptable salt thereof for use in a method of treatment of fatty liver disease or HCC.

## BRIEF DESCRIPTION OF THE DRAWINGS

Fig. 1 is a bar graph demonstrating the log2 fold changes of the top three connected genes in cluster 2 in early and moderate NAFLD patients as well as in mice fed by HSD and HFD compared to their corresponding controls. An asterisk indicates a statistical significance difference (P adj. < 0.05).

Fig. 2A-C are bar plots showing the drug effects on protein expression of HepG2 and steatosis-induced HepG2, normalized by β-actin. Fig. 2D is a bar plot showing the drug effects on triglyceride contents of

steatosis-induced HepG2.

Fig. 3 is a table showing scoring of histopathological and immunohistochemical findings in liver tissues.

Fig. 4 is a bar plot showing the result of an MTT assay (treatment of liver cancer cells).

Fig. 5 is a table showing the dosage of the drugs used in an in vitro experiment.

Fig. 6 is a scheme for the synthesis of JNK-IN-5A (also referred to as JNK Inhibitor IX).

## DETAILED DESCRIPTION

It is shown herein that JNK-IN-5A (N-(3-cyano-4,5,6,7-tetrahydrobenzo[b]thien-2-yl)-1-naphthalenecarboxamide; formula (I)) may be used for treatment of fatty liver disease and HCC. JNK-IN-5A is sometimes referred to as JNK Inhibitor IX. A synthetic scheme for JNK-IN-5A is shown in Fig. 6. JNK-IN-5A is also commercially available.

Accordingly, there is provided as a first aspect of the present disclosure a compound of formula (I) a pharmaceutically acceptable salt thereof for use in a method of treatment of a fatty liver disease or hepatocellular carcinoma (HCC).

Formula (I) is shown above.

In one embodiment, the method of treatment of the first aspect comprises oral administration of the compound. The skilled person is familiar with suitable formulation strategies for facilitating such administration. The references to the methods of treatment by therapy in this description are to be interpreted as references to compounds, pharmaceutical compositions and medicaments of the present invention for use in those methods.

In an embodiment, the method of treatment of the first aspect comprises detection of overexpression of PKLR in the liver of the subject to be treated prior to administration of the compound to the subject. Overexpression may for example be a higher level of expression than a reference level corresponding to an average level of expression in healthy liver tissue. Alternatively, overexpression may be a higher level of expression than a reference level corresponding to an average level of expression in NAFLD or HCC liver tissue.

In a preferred embodiment, the fatty liver disease is non-alcoholic fatty liver disease (NAFLD). Said NAFLD may have progressed to non-alcoholic steatohepatitis (NASH).

The subject of the treatment of the first aspect is preferably a human.

As a second aspect of the present disclosure, there is provided a method of treatment of fatty liver disease or HCC in a subject in need thereof, said method comprising administering to the subject a therapeutically effective amount of a compound of formula (I).

The embodiments and examples of the first aspect apply to the second aspect *mutatis mutandis.*

As an alternative aspect of the present disclosure, the compound BX-912 or a pharmaceutically acceptable salt thereof is provided for use in a method of treatment of a fatty liver disease (e.g. NAFLD or NASH) or hepatocellular carcinoma (HCC). Such treatment may comprise oral administration of BX-912. The embodiments and examples of the first aspect apply to the alternative aspect *mutatis mutandis.*

The formal name of BX-912 is N-[3-[[5-bromo-4-[[2-(1H-imidazol-5-yl)ethyl]amino]-2-pyrimidinyl] amino]phenyl]-1-pyrrolidinecarboxamide.

## EXAMPLES

### Methods

Pkl KO mouse model

The Pkl KO mice as well as the wild-type ones were purchased from Applied StemCell company. The company generated a knockout (KO) model in mouse Pklr gene. The model specifies a Guanine (G) deletion immediate downstream of the ATG start codon in mouse Pklr L-isozyme, not R-isozyme.

Mouse animal studies

In the first experiment, twelve male C57BL/6J mice were fed a standard mouse chow diet (Purina 7012, Harlan Teklad) and housed in a 12-h light-dark cycle. From the age of 8 weeks, the mice were then divided into three groups of 4 mice fed with chow diet, high-sucrose diet and high-fat diet, respectively, for three weeks. At the age of 11 weeks, all mice were sacrificed and liver necropsy were taken for RNA sequencing and lipid quantification.

In the second experiment, eight male C57BL/6J wild-type mice and eight $Pklr^{-/-}$ mutant mice were fed a standard mouse chow diet (Purina 7012, Harlan Teklad) and housed in a 12-h light-dark cycle. From the age of 8 weeks, both the wild-type and mutant mice were then divided into two groups of 4 mice fed with chow diet, high-sucrose diet, respectively, for another 8 weeks. At the age of 16 weeks, all mice were sacrificed and necropsies from liver, muscle, WAT and heart tissue were taken for RNA sequencing and lipid quantification.

All mice were housed at the University of Gothenburg animal facility (Lab of Exp Biomed) and supervised by university veterinarians and professional staff. The health status of the mice was constantly monitored according to the rules established by the Federation of European Laboratory Animal Science Associations. The experiments were approved by the Gothenburg Ethical Committee on Animal Experiments.

Rat animal studies

*In vivo biosafety studies.* Healthy Sprague Dawley male rats (12 weeks old, 250-280 g) were obtained from Atatürk University Experimental Research Center (ATADEM, Erzurum Turkey) and housed under standard environmental conditions (temperature 20 °C-25 °C, humidity 50 ± 20% and 12-hour light/dark diurnal cycle). Food and water were available ad libitum. Rats were randomly separated into one control group and two experimental groups (30 mg/kg/day BX-912 or JNK-IN-5A, n=5). BX-912 and JNK-IN-5A were dissolved in DMSO and orally administrated using oral gavage for 7 days. The control group were received the same amount of vehicle solution only.

After 7 days, rats were anaesthetized, and blood samples were collected from the abdominal aorta for biochemical and haematological analysis. The plasma was separated and stored at -80 °C for further analysis. Major organs such as heart, kidney, liver, muscle, adipose, intestine (duodenum, ileum, jejunum), pancreas, colon, and stomach were collected from all the animals for further analysis. Selected organs were fixed in 10% neutral buffered formalin for histopathological examination.

In addition to sampling for biochemical and haematological analysis, one drop of blood samples was obtained from the animals for evaluating micronucleus frequencies as genotoxicity endpoint at the end of the experiments. Three different smears of each rat were prepared using pre-coded and cold slides, then the slides air-dried at room temperature, and fixed in ethanol for 10 min, and stained with Giemsa. The frequencies of micronucleated polychromatic erythrocytes (MNPCEs) were analysed according to previously suggested approach using a light microscope. The incidence of MN-PCEs was determined by scoring a total of 3000 PCE per animal and results were expressed as % MN (Solórzano-Meléndez et al., 2021).

*In vivo efficacy studies.* After 1-week acclimation period, rats were randomly separated into two groups: control group (n=5) and high sucrose group (n=20). The control group was fed with a standard diet while the others consumed high sucrose diet (HSD). Sucrose was purchased from Sigma Chemical Co. (St Louis, MO, USA) and supplied at the dose of 10% in the drink water for two weeks.

After 2 weeks, five rats in the HSD group were randomly sacrificed for conformation of NAFLD model. Then, the remaining rats in the HSD group were separated into three sub-groups (n=5): HSD group, HSD plus BX-912 group and HSD plus JNK-IN-5A group. BX-912 and JNK-IN-5A were dissolved in DMSO and were orally administrated using oral gavage for one week (30 mg/kg/day). The HSD group were treated with vehicle solution only.

At the end of the experiment, rats were sacrificed after being anesthetized. Blood samples were collected from the abdominal aorta and centrifuged at 8000 rpm for 15 min at 4 °C. The plasma was separated and stored at -80 °C until further analysis. Other internal organs, including the heart, adipose tissues, liver, kidney, muscle, intestine (duodenum, ileum, jejunum), pancreas, colon and stomach were immediately removed and then snap frozen in liquid nitrogen and stored at -80°C; the liver was also fixed in 10% formaldehyde for histopathological examination.

The biosafety and efficacy studies were approved by The Ethics Committee of Atatürk University, and all experiments were carried out in accordance with relevant guidelines and regulations for the care and use of laboratory animals. Animal Experiments Local Ethics Committee of Atatürk University approved the experimental procedure described in this study (Approval Date: 27.08.2020; Approval Number: 42190979- 000-E.2000208344).

*Histopathological examination.* The liver tissues were fixed in 10% buffered formaldehyde solution. After fixation, the tissues were passed through graded alcohol and xylene series and embedded in paraffin blocks. 5 micrometer thick sections were taken serially from the paraffin blocks. Histopathological changes were evaluated by performing hematoxylin eosin staining on the sections taken. Sections were evaluated according to histopathological findings as none (-), mild (+), moderate (++) and severe (+++).

*Immunohistochemical examination.* After deparaffinization, the slides were immersed in antigen retrieval solution (pH 6.0) and heated in microwave for 15 min to unmask the antigens. The sections were then dipped in 3% $H_2O_2$ for 10 min to block endogenous peroxidase activity. Protein block was dropped onto the sections, washed with PBS for 10 min. Primary antibodies (8-OH-dG, Cat No: sc-66036, Diluent Ratio: 1/100, Santa Cruz) were prepared and applied according to the usage conditions. Expose mouse and rabbit specific HRP/DAB detection IHC kit were used as follows: sections were incubated with goat anti-mouse antibody, then with streptavidin peroxidase, and finally with 3,3' diamino benzidine + chromogen. Slides were counter stained with hematoxylin. Immunoreactivity in the sections were graded as none (-), mild (+), moderate (++) and severe (+++).

## Cell experiments

WT HepG2 was included in this study and cultured by Roswell Park Memorial Institute (RPMI, R2405, Sigma Aldrich) 1640 Medium with 10% fetal bovine serum (FBS) and 1% penicillin - streptomycin (PS). To induce a steatosis model into WT HepG2 cells, high glucose Dulbecco's Modified Eagle Media (DMEM, D0822/D5671, Sigma-Aldrich) containing 10% FBS and 1% PS was used as medium for one week, together with 10μg/mL insulin and 10μM T0901317. All of cells were incubated in the incubator with 5% $CO_2$ and 37°C (Thermo SCIENTIFIC). Fresh medium was provided every two days.

Nine drugs were tested in this study and all of

them were bought from commercial companies. All the drugs were diluted in Dimethyl sulfoxide (DMSO, 41639, Sigma-Aldrich) and stored in -20°C.

As for drug-screening, different numbers of HepG2 cells were seeded for distinct experiments. More in details, 40,000 cells and 500,000 cells were used for 2-day-drug treatment in 96-well-plate and 6-well plate, respectively, while 80,000 cells and 300,000 cells were used for one-week drug treatment in 96-well plate and 6-well plate, respectively. Detailed drug dosages could be found in Fig. 5. Fresh medium was provided every two days.

Transcriptomic data from human patients

The key genes identified in mouse fed HSD were validated by performing differential expression analysis for the publicly available human patient datasets where the study details and its characteristics are also reported (Govaere et al., 2020).

RNA extraction and sequencing

Total RNA was isolated from homogenized heart tissue using RNeasy Fibrous Tissue Mini Kit (Qiagen). cDNA was synthesized with the high-capacity cDNA Reverse Transcription Kit (Applied Biosystems) and random primers. mRNA expression of genes of interest was analyzed with TaqMan real-time PCR in a ViiA™ 7 system (Applied Biosystems). RNA sequencing library were prepared with Illumina RNA-Seq with Poly-A selections. Subsequently, the libraries were sequenced on NovaSeq6000 (NovaSeq Control Software 1.6.0/RNA v3.4.4) with a 2x51 setup using 'NovaSeqXp' workflow in 'S1' mode flow cell. The Bcl was converted to FastQ by bcl2fastq_v2.19.1.403 from CASAVA software suite (Sanger/phred33/Illumina 1.8+ quality scale).

Lipid quantification

Lipids were extracted as described previously (Lofgren et al, 2012). Internal standards were added during the extraction. Lipids were analysed using a combination of HPLC and mass spectrometry as described (Stahlman et al, 2013). Briefly, straight-phase HPLC was used to purify ceramides (CER). Cholesteryl ester (CE), triacylglycerol (TAG), phosphatidylethanolamine (PE), phosphatidylcholine (PC), and sphingomyelin (SM) were quantified using a QTRAP 5500 mass spectrometer (Sciex, Concord, Canada) equipped with a robotic nanoflow ion source, TriVersa NanoMate (Advion BioSciences, Ithaca, NJ). CER were analysed using reversed-phase HPLC coupled to a triple-quadrupole Quattro Premier mass spectrometer (Waters, Milford, MA, USA).

Western blot

Protein content levels were detected by western blot. After the respective treatment of drugs, the cell pellets were lysed in cell lytic M, followed by protein extraction from supernatant (10min, 13,000r/min centrifuge). Extracted cell lysate was stored in -20°C. Proteins were quantified through Bovine albumin serum (BAS) standard curve with protein assay dye reagent (BIO-RAD). The proteins were separated by SDS-PAGE with 100v in 90 min by mini-protein TGX precast gels (4-15%, 15 wells, 15μl/well, BIO-RAD), and then transfer to Mini PVDF transfer package through transfer system (Transfer-Turbo, BIO-RAD). After 30min blocking with 5% skim milk in cold room (4°C) and washing with TBST, membrane was cut by the size of interested proteins. Primary antibodies were added into the corresponding membrane sections for overnight binding. Then the membranes were washed with TBST, and the secondary antibodies were incubated for 1h in cold room. The following antibody were used: (Goat pAb to Rb Ig, ab205718, abcam; Rb pAb to beta-actin, ab8227, abcam; anti-pklr, HPA006653, SIGMA; Rb pAb to fatty acid synthase, ab99539, abcam). The bindings were imaged by immunostaining and machine (ImageQuant LAS 500). All antibodies were purchased from Abcam online, and β-actin was used as global normalized protein. After measurement, the strength of signal for each protein was quantified by ImageJ. The background noise was removed, and all results were normalized by the levels of β-actin.

Cell viability assay & Triglyceride assay for in vitro experiment

Cell viability and lipids content were detected on the same batch of cells. After one-week treatment of drugs in steatosis model, drug cytotoxicity was determined by Cell Counting Kit 8 (CCK-8, Sigma-Aldrich) and triglyceride levels were detected by Triglyceride Quantification Assay (TAG) kit (Abcam, ab65336). The absorbance was then measured at 450 nm and 570 nm, respectively. To normalize triglyceride content level, average values of cell viability were applied.

Transcriptomics data

The raw RNA-sequencing results were processed using Kallisto (Bray et al., 2016) with index file generated from the Ensembl mouse reference genome (Release-92) (Zerbino et al., 2018). The output from Kallisto, both estimated count and TPM (Trancript per kilobase million), were subsequently mapped to gene using the mapping file retrieved from Ensembl BioMart website, by filtering only protein coding genes and transcripts. Genes with mean expression less than 1 TPM in each condition were filtered. For data exploration, PCA from sklearn package (https://dl.acm.org/doi/10.5555/1953048.2078195pi) was used in Python 3.7 and TPM values were used as the input.

Subsequently, differential gene expression analysis was performed using DESeq2 package in R (Love et al., 2014). To define a gene as differentially expressed (DEGs), a gene has to fulfil a criterion of FDR < 5%. The results of differential expression analysis were then used for functional analysis.

Functional analysis was performed using the R package PIANO (Varemo et al., 2013). As the input, the fold changes and p-values from the DESeq2 and KEGG pathways gene-set collections from Enrichr were used. To define a process or pathway as significant, a cut off of FDR < 10% was used for the distinct direction of PIANO (both up and down).

Co-expression analysis

The co-expression network was generated by generating gene-gene Spearman correlation ranks within a tissue type, using spearmanr function from SciPy (Virtanen et al., 2020) in Python 3.7. Using the same environment, multiple hypothesis testing was performed using Benjamini-Hochberg method from statsmodels. Correlation data were filtered with criterion of adjusted p-value < 5%.

The top 5% of filtered correlation results were then loaded into iGraph module (https://cran.r-project.org/web/packages/igraph/citation.html) in Python 3.7 as an unweighted network. To find the subnetworks, the Leiden clustering algorithm (Traag et al., 2019) was employed with the ModularityVertexPartition method. Each cluster was analysed by using Enrichr (Chen et al., 2013; Kuleshov et al., 2016) to get the enriched KEGG pathways. Criterion FDR < 5% were used to find the significantly enriched terms. Clusters with less than 30 genes was discarded, to be able to get significant functional analysis results.

Identification of Pklr gene signature

The Pklr signature was determined by meta-analysis of three individual RNA-seq datasets. The first two datasets were obtained from a previous study (Liu et al., 2019) where siRNA/plasmid was used to inhibit and over-express Pklr in HepG2 cell line, respectively. Raw sequencing data was mapped by Kallisto based on GRCh38 (gencode v23). Differential expression analysis with R package DESeq2 was performed for the two datasets. The results of DEGs were recognized as gene signature, which respectively referred to Pklr inhibition signature and Pklr over-expression signature hereafter. Next, RNA-seq data of liver biopsies from 110 healthy donors generated by the Genotype-Tissue Expression (GTEx) project was collected. After removing low expressed genes (TPM<1), the spearman correlation coefficient between Pklr and other genes, referred to Pklr co-expression signature, was calculated. In summary, three Pklr-related gene signatures were detected, namely Pklr co-expression signature (N = 11733), Pklr inhibition signature (N = 16399) and Pklr over-expression signature (N = 14903).

The next step was to generate a Pklr consensus signature by aggregating the three-candidate gene lists. First of all, genes were defined as Pklr positively related genes if they met three conditions at the same time: positive log2 fold change values in Pklr over-expression signature group; negative log2 fold change values in Pklr inhibition signature group; positive correlation coefficient in Pklr co-expression signature. The definition of Pklr negatively correlated genes was the opposite. These Pklr positively/negatively correlated genes generated a list of Pklr consensus correlated genes (N=4994).

To rank these consensus genes by the level of relationship with Pklr in a uniform standard, a combined z-score was calculated for them by aggregating the P-values from the three lists of Pklr signatures. Specifically, for each list of P-values, the P-Values (two-tailed significance test) were transformed to z-score as follows:

$$p = 2\Phi(-|Z|)$$

where $\Phi$ is the standard normal cumulative distribution function (Lee et al., 2016).

Then a combined z-score was calculated for 4998 combined PKL correlated genes. The equation was as follows:

$$Z_{IVW} = \frac{\bar{X}}{SE(\bar{X})} = \frac{\sum W_i X_i}{\sqrt{\sum W_i}}$$

where X is the z score from three independent results and W is the weight (set as 1 for all of three results) and IVW is the Inverse variance-weighted (IVW) average method (Lee et al., 2016).

Consequently, the 4998 Pklr consensus correlated genes could be ordered by the aggregated z-scores, and these z-scores were defined as the final Pklr consensus signature. Notably, z-scores of negatively correlated genes were labelled as negative values and vice versa in order to reflect the direction of genes.

Drug repositioning

The fifth level of LINCS L1000 phase 2 data were downloaded (GEO: GSE70138) and used for detecting the connectivity of small molecules and gene signatures. HepG2 cell line data was specifically parsed from the whole file, containing differential expressed gene signatures after small molecules' treatment. Then the Spearman correlation coefficient was calculated between the Pklr consensus signatures and these drug-perturbed gene signatures, focusing on the 3698 pair-wised genes. P-Values were adjusted based on the

Benjamini-Hochberg method. Drugs were ranked by r value, and the top 10 drugs were considered potential Pklr inhibitors (r < -0.4, FDR < 1E-95). In total, there were 10 drugs extracted, and nine of them could be purchased and were hence selected for wet lab validation.

Data and code availability

All raw RNA-sequencing data generated from this study can be accessed through accession number GSE. Codes used during the analysis are available on https://github.com/sysmedicine/pklr

MTT assay

HepG2 cells were seeded into a 96well plate at 20,000 cells per well as hexaplicated. Next day, treatment of the cells with 10μM drugs (BX-912 and JNK-IN-5A) was started. The treatment lasted for two days. Cell viability was measured using MTT assay. Cells were incubated with 5mg/ml MTT 3-4 5-DIMETHYLTHIAZ (M6494 Thermo fisher) in PBS for 2 h. Microplate reader measured cell viability O.D at 570 nm.

**Results**

Modulation of PKLR with different diets

To investigate the effects of different diets on the expression of PKLR and other functionally associated protein-coding genes, three groups of 8 weeks old C57BL/6J male mice were fed with CHOW, HSD and HFD, respectively, for three weeks. After the three weeks, the mice were sacrificed and liver samples were collected to examine the liver fat content and analyse global gene expression profiling. Liver triglycerides (TGs) and cholesterol esters (CEs) levels were measured and it was found that the level of TGs and CEs are significantly increased in the liver of mice fed with HFD and HSD compared to the liver of mice fed with CHOW ($P < 0.05$).

To reveal the underlying molecular mechanisms involved in the accumulation of fat in the liver with HFD and HSD diets, mRNA was extracted from the liver tissues and transcriptomics data was generated using RNA sequencing (see Method). A principal component analysis (PCA) was performed based on liver transcriptomics data and it was found that HFD and HSD are deviated from CHOW along with principal component 1 (PC1) and 2 (PC2), respectively, suggesting that HFD and HSD diets induced different responses in the liver. First, the key genes deviating the HSD from CHOW were investigated and it was found that Pklr, Acaca, Acacb, Sed, Acly and Fasn are the most critical genes leading to separation on PC1. These genes are involved in the DNL, which is known to be associated with NAFLD development. Interestingly, it was also found that Pnpla3, which is known to be related to NAFLD progression, is among the top separating genes on PC1 (absolute loading>0.2). Next, the key genes deviating the HFD from CHOW were investigated, and it was found that Mvd, Idi1, Fdps and Nsdhl, involved in cholesterol metabolism, Acnat2 and Cyp7a1, involved in bile secretion and Acot1and Acaa1b, involved in the fat oxidation, are leading to separation on PC2 (absolute loading>0.2).

Second, the differentially expressed genes (DEGs) in the liver of HFD-fed and HSD-fed mice compared to CHOW-fed mice were identified, and it was found that 278 and 1302 genes, respectively, are significantly differentially expressed in mice fed with HFD and HSD (P adj. < 0.05). A recent study reported that the expression of three genes, namely PKLR, PNPLA3 and PCSK9, are significantly positively associated with the expression of FASN, hepatic DNL and progression of NAFLD (Lee et al., 2017). The expression of these genes was checked and it was found that Pklr, Pnpla3, Pcsk9 and Fasn are significantly upregulated in the liver of HSD-fed mice, but not in the liver of HFD-fed mice, when compared to CHOW-fed mice. These findings indicate that the expressions of Pklr and other functionally associated genes are modulated by HSD, which also induces hepatic DNL and liver fat in mice.

Third, the changes in the global expression of the genes was investigated based on KEGG pathways (Kanehisa et al., 2021) using the PIANO toolbox (Varemo et al., 2013). The activated pathways induced by HSD and HFD compared to CHOW were identified. In HFD-fed mice, 130 and 148 genes were found to be significantly up- and down-regulated, respectively. The up-regulated genes are enriched in pathways associated with the biosynthesis of unsaturated fatty acids and inositol phosphate metabolism. The down-regulated genes are enriched in pathways related to protein processing, transcription, glycolysis, fatty acids beta-oxidation and terpenoid/steroid metabolism. In HSD-fed mice, 634 and 668 genes are found to be significantly up- and down-regulated, respectively. The up-regulated genes are enriched in many metabolic pathways associated with glycolysis, fatty acid metabolism, glycan metabolism, steroid biosynthesis, and pathways related to protein processing and insulin response. The down-regulated genes are enriched in amino acids metabolism, fatty acids beta-oxidation. Notably, it was found that both HFD and HSD diets induced the downregulation of glycine, serine and threonine metabolism and cysteine and methionine metabolism. These pathways are essential for synthesising glutathione, which is critical for NAFLD progression based on a previous study (Mardinoglu et al., 2017).

Next, it was found that the significantly enriched focal adhesion and ECM-receptor interaction pathways related to cell morphogenesis and extracellular interaction are associated with the up-regulated genes in both groups. These two pathways have been associated with the development of hepatic fibrosis or cirrhosis (Zhang and Zhang, 2020; Zhao et al., 2017), and their elevation, thus, indicated the potential advert effect of both HFD and HSD. In addition, it was observed that some other enriched morphogenesis and extracellular interaction related pathways are associated explicitly with the upregulated genes in HSD-fed and HFD-fed mice, respectively, implicating that these two diets may induce morphological changes with different mechanisms. Moreover, it was found that both mice groups exhibited attenuated hepatic expression of complement and coagulation cascades pathway, suggesting the dysregulated immune responses induced by HFD and HSD.

Finally, the alteration of signalling pathways induced by HFD and HSD were analysed. It was observed that up-regulated genes in both HFD and HSD are enriched in key signalling pathways such as MAPK, PI3K-Alt, TGF-beta and Wnt signalling pathways. Notably, several interesting signalling pathways associated with only up-regulated genes in HFD were observed, which further demonstrated the difference between the two diets. For instance, the PPAR signalling pathway is associated explicitly with the up-regulated genes in the HFD-fed mice group. It was found that the expression of several key player genes in PPAR signalling pathways, including Fabp2 (the most up-regulated gene), Slc27A2, Pparg, Cd36, Gk, Scd2 and Sorbs1 are significantly elevated. Cd36 is a fatty acid translocase, and its elevation suggested an increased hepatic fatty acid influx (Koonen et al., 2007). Although Pparg is primarily expressed in adipose tissue, several studies have also reported that hepatic Pparg expression is up-regulated in NAFLD to activate the adipogenic pathway to store lipid in the liver (Lee et al., 2018). Therefore, it was observed that the up-regulation of the PPAR signaling pathway could be a hepatic regulatory response to HFD.

Based on KEGG pathway analysis, the different hepatic responses to HSD and HFD were briefly summarized. The HFD-fed mice exhibited an elevated hepatic intake of free fatty acids (FFAs) through Cd36 to oxidize the excessive fat in the diet, and the FFAs influx activates Pparg, which sequentially enhanced the lipogenesis from FFAs. HFD also suppressed steroid and terpenoid backbone biosynthesis pathways and protein processing related pathways. The former may link to a dysregulated steroid hormones homeostasis (Charni-Natan et al., 2019), while the latter might suggest a decreased lipoprotein metabolism. A significant down-regulation of Apom was also observed (P adj. $< 0.05$). On the other hand, HSD-fed mice induced glycolysis and DNL leading to fat accumulation in the liver and insulin resistance. It was also observed that up-regulated genes in HSD are significantly enriched in insulin signaling, glucagon signaling pathways and insulin resistance pathways. In addition, it was found that the downregulated genes are enriched in the BCAAs degradation pathway, which is associated with the progression of NAFLD and type 2 diabetes (T2D) (Chen and Yang, 2015; Gaggini et al., 2018) and indicates less activated oxidative phosphorylation and peroxisome beta-oxidation, as was also observed in the enrichment analysis.

Revealing the dietary response using CNs

Liver-specific CNs were generated and network-based analysis was performed to elucidate the functional and pathogenic roles of genes in the mouse liver. All genes pairs with significant correlations were selected as edges to construct the CNs and Leiden clustering algorithm (Traag et al., 2019) was employed to define the structure of the CN by maximizing the modularity scores. Next, functional enrichment analysis was performed to identify the specific functions associated with each cluster by utilizing the Enrichr algorithm (Chen et al., 2013; Kuleshov et al., 2016) and the KEGG pathways that are significantly enriched in each gene cluster (FDR $< 0.05$) were summarized. It was found that two of the largest clusters, cluster 0 and 2, are enriched in mainly metabolic pathways. Specifically, genes in cluster 0 are enriched in amino acids metabolism and fatty acid degradation pathways, while genes in cluster 2 are enriched in glycolysis, oxidative phosphorylation, fatty acid biosynthesis, glycerolipid and cholesterol metabolic pathways. These highlighted metabolic pathways had a critical role in response to the dietary changes in mice liver. In addition, the DEGs in HFD-fed and HSD-fed mice were respectively superimposed to the CNs to identify the components of clusters affected due to dietary changes. It was found that cluster 0, 1, 2, 4, 6, 7 and 8 are enriched with significantly changed DEGs in at least one direction (hypergeometric test, P adj. $< 0.05$). Intriguingly, it was observed that cluster 2 includes Pklr, Fasn and Pnpla3 and is enriched with both up-regulated genes in the HSD condition and down-regulated genes in the HFD condition.

Considering that most of the enriched pathways of cluster 2 are associated with NAFLD, it was observed that this cluster is the most relevant for studying the effect of different diets on NAFLD progression. The most central genes in cluster 2 were investigated based on their degrees. Interestingly, Pklr and Pnpla3 both appeared among the top 20 genes with the highest degree in this cluster, confirming their key role in controlling glucose and fatty acid metabolism. Notably, the degree of Pklr is ranked third within this cluster, following Elovl6 and Gpam, which are involved in fatty acid elongation and glycerolipid metabolism, respectively. In addition, these top three genes had degrees that are distinctly higher than other nodes. Therefore, it was concluded that these three genes are the key hubs in this cluster.

To investigate the association of the top degree genes in the progression of NAFLD, the genome-wide hepatic mRNA expression in patients with early and moderate NAFLD was quantified (Govaere et al., 2020) and compared to that of normal subjects using differential expression analysis. As expected, Elovl6, Gpam and Pklr showed significant up-regulation in both early and moderate NAFLD patients, emphasizing their essential roles in NAFLD progression (Fig. 1). In addition, their hepatic expression changes in both HFD-fed and

HSD-fed mice compared to CHOW-fed mice were examined and it was found that their expression is only significantly up-regulated in HSD-fed mice (Fig. 1). Moreover, the overlap of the up-regulated genes in these four groups were studied in a pair-wised manner and it was found that only up-regulated genes in the HSD group, but not those in the HFD group, are significantly intersected with those up-regulated in the two NAFLD groups (hypergeometric P <0.05).

Finally, a subnetwork was extracted based on these three key hub genes and their top 20 correlated neighbors in cluster 2. The genes that are up-regulated in HSD-fed or HFD-fed mice as well as in early and moderate NAFLD patients were highlighted. It was found that most of the genes in this central subnetwork of cluster 2 are significantly upregulated in HSD-fed mice compared to CHOW-fed mice. It was observed that many of the genes in this subnetwork are significantly elevated in the two NAFLD patient groups, while none showed a significant increase in HFD-fed mice. The expression changes of all genes in this subnetwork in the four groups were demonstrated and it was shown that only the HSD group showed a similar expression pattern to the NAFLD patients. Taken together, these results suggested that HSD induces hepatic expression changes in mice in a way that is much more similar to the changes observed in NAFLD patients than the expression changes induced by HFD.

<u>The effect of PKL KO on liver</u>

It has previously been reported that the PKL is a potential target for NAFLD treatment (Lee et al., 2017; Liu et al., 2019). To the knowledge of the present inventors, the systemic effect of PKL inhibition has not been studied *in vivo.* In this context, a knockout (KO) model in mouse Pklr gene was generated and it was verified that targeting Pkl could attenuate NAFLD progression. The model specifies a Guanine (G) deletion immediate downstream of the ATG start codon in mouse Pklr L-isozyme, not R-isozyme. Four groups of 8 weeks old Pkl KO and control C57Bl/6J (Ctrl) mice were fed with either HSD or CHOW for eight weeks. No significant phenotypic differences or noticeable illness in Pkl KO mice compared to the control group was observed. Next, the liver and plasma lipid levels in all four groups of mice was carefully examined and it was found that HSD-fed mice developed significant liver fat compared to CHOW-fed mice. The Pkl KO mice were well tolerant of the HSD and Pkl KO HSD-fed mice had significantly lower liver TG levels than the Ctrl HSD-fed mice. It was found that the liver levels of ceramide, lactosylceramide and sphingomyelin were significantly decreased in Pkl KO mice compared to the Ctrl HSD-fed mice. It was also found that phosphocholine level was slightly increased in Pkl KO HSD-fed mice compared to the Ctrl HSD-fed mice.

To systematically study how the Pkl KO prevent the development of liver fat in mice fed with HSD, liver tissue samples were collected from these four groups of mice and RNA-seq data was generated. Firstly, the hepatic Fasn expression was examined and found to be significantly elevated in Ctrl HSD-fed mice but not in the Pkl KO HSD-fed mice. Analysis indicated that Pkl KO inhibits the hepatic DNL in HSD-fed mice. It was found that there is no significant difference in Fasn expression between the Pkl KO CHOW-fed mice and Ctrl CHOW-fed mice, and this is also supported by the measurement of comparable liver TG levels between these two groups of mice.

Next, focus was put on two comparisons, namely Ctrl HSD-fed vs Ctrl CHOW-fed mice and Pkl KO HSD-fed vs Ctrl HSD-fed mice and the transcriptomic changes between high liver fat vs normal as well as Pkl KO treatment vs high liver fat, respectively, were analyzed. Differential expression analysis and pathway enrichment analysis using KEGG pathways were performed. 957 DEGs between Ctrl HSD-fed vs Ctrl CHOW-fed mice and 90 genes in Pkl KO HSD-fed vs Ctrl HSD-fed mice were identified. Notably, it was found that 58 DEGs are shared between these two groups and a significant transcriptomic change in the opposite direction (hypergeometric P = 0) was shown. Functional enrichment analysis was performed using these 58 overlapped DEGs and it was found that they are only significantly enriched in circadian rhythms (P adj. < 0.05), which suggests the Pkl KO reversed the dysregulation of circadian rhythms induced by HSD. Notably, the expression level of these DEGs in the circadian rhythm's pathway is not significantly altered in the Pkl KO mice compared with the wild-type mice fed with CHOW (P adj. > 0.05), implying that the KO does not change the normal circadian regulation in a healthy state. The transcriptomic changes' landscape was analyzed and an apparent negative correlation between the fold changes of the transcriptomic expression between these two comparisons (Spearman corr. = -0.398) was found. Also, the PIANO toolbox was employed to investigate the pathway level transcriptomic changes based on the KEGG pathways. The pathway-level changes identified in HSD vs CHOW are very similar to those found in the 3-week comparison, including the up-regulation of glycolysis, insulin signaling pathway, protein processing in ER, fatty acid metabolism, steroid and terpenoid metabolism and downregulation of amino acid metabolism and transcription. Although a fewer number of pathways showed significant alteration in the Pkl KO HSD-fed compared to Ctrl HSD-fed mice, the transcriptomic expression of several of these pathways, such as protein processing in ER, glycolysis, insulin signaling pathway, steroid biosynthesis, ribosome and spliceosome pathways, are significantly reversed after Pkl KO.

Finally, liver-specific CN was constructed using the samples from Ctrl and Pkl KO mice (n = 12), the co-expression gene clusters were identified using the same method as mentioned above, and the functional relations of genes were elucidated. As a result, ten clusters of

genes that are significantly co-expressed (n>200) were identified, and six of them, namely cluster 0, 2, 4, 6, 8 and 9, are enriched with DEGs identified in Ctrl HSD-fed vs Ctrl CHOW-fed and Pkl KO HSD-fed vs Ctrl HSD-fed mice in at least one direction (hypergeometric $P<0.05$). Also, the KEGG pathways enriched in each gene cluster ($FDR<0.05$) were summarized, and focus was put on cluster 8, which includes the key DNL genes, such as Fasn, Pnpla3, Elovl6 and Gpam as well as on cluster 4 and cluster 9, which are both enriched with DEGs in two different comparisons. Genes in cluster 8 are significantly overlapped with the up-regulated DEGs in Pkl KO HSD-fed vs Ctrl HSD-fed mice. These genes are enriched in glutathione metabolism, pyruvate metabolism, steroid biosynthesis TCA cycle, fatty acid biosynthesis and PPAR signaling pathway. The functional association of this cluster was expected as several DNL marker genes are included in this cluster. It was however found that Pklr, which was closely co-expressed with key DNL genes, was moved from cluster 8 to cluster 4. Genes in cluster 4 are significantly overlapped with the up-regulated DEGs in Ctrl HSD-fed vs Ctrl CHOW-fed mice and the down-regulated DEGs in Pkl KO HSD-fed vs Ctrl HSD-fed mice. These genes are enriched in endocytosis, proteasome, protein processing in ER, N-glycan biosynthesis and PI3K-Akt signaling pathway. The analysis suggested that the functions related to this cluster is up-regulated in the disease model, and the up-regulation is attenuated after Pkl KO. Considering Pklr is included in cluster 4, it also suggested that Pklr has a close relation with protein processing and PI3K-Akt signaling. Genes in cluster 9 are significantly overlapped with the down-regulated DEGs in Ctrl HSD-fed vs Ctrl CHOW-fed mice and the up-regulated DEGs in Pkl KO HSD-fed vs Ctrl HSD-fed mice. These genes are enriched in complement and coagulation cascades, tryptophan metabolism, BCAAs degradation, fatty acids degradation and retinol metabolism pathways. The analysis suggested that the functions related to this cluster are down-regulated in the disease model, and the down-regulation is attenuated after Pkl KO. In summary, the CN based analysis indicated that the Pkl KO mainly regulated the pathways related to inflammation, protein processing, BCAA degradation, DNL and fatty acids degradation, which are closely related to the pathogenesis of NAFLD.

Investigating NAFLD progression and Pkl KO effect in other metabolic tissues

It has been reported that the progression of NAFLD is associated with extrahepatic tissues, such as WAT, muscle and heart tissues (Haas et al., 2016). Hence, it is vital to analyse the global transcriptomics changes in multi-tissue context to study the molecular differences during NAFLD progression and Pkl KO. WAT, muscle and heart tissue samples from the 8-week Ctrl and Pkl KO mice fed with both CHOW and HSD were obtained and RNA-Seq data was generated. First, differential expression analysis between Ctrl HSD-fed vs Ctrl CHOW-fed mice and Pkl KO HSD-fed vs Ctrl HSD-fed mice in these three tissues was performed and a comparison to the DEGs in the liver was made. Interestingly, the largest number of DEGs was observed for WAT in all comparisons (1026 and 238, respectively), followed by the muscle (236 and 5, respectively) and heart (32 and 2, respectively). The analysis suggested that WAT is the most responsive extrahepatic tissue to HSD as well as the Pkl KO among the three tissues, and the other tissues have a relatively mild response.

Focus was on investigating the changes in WAT. The overlapped DEGs between the two comparisons (Ctrl HSD-fed vs Ctrl CHOW-fed and Pkl KO HSD-fed vs Ctrl HSD-fed) were checked and it was found that 58 genes are significantly reversely altered in these two comparisons (hypergeometric $P = 0$), which is very similar to what was observed in the liver. In addition, although not statistically significant, the top enriched KEGG pathways of these 58 genes are circadian entrainment and circadian rhythm ($FDR = 0.17$), which is also in excellent agreement with the 58 reversed changed DEGs identified in the liver. This might indicate that circadian regulation played an important role in the whole-body response to Pkl KO. Moreover, a negative correlation between the fold changes of the gene expressions was observed between these two comparisons (Spearman corr. = -0.436). The changes in muscle and heart tissues were also investigated. Notably, the DEGs are also significantly reversely overlapped in muscle and heart tissues (hypergeometric $P < 0.05$) and their gene expression changes also showed negative correlations. Taken together, it was observed that the hepatic Pkl KO also partly reversed the transcriptomic changes in extrahepatic tissues induced by HSD.

Next, the pathway level changes were investigated using the DEGs in all three tissues using PIANO and KEGG pathways in the same way as in the liver tissue. In WAT, significant up-regulation of protein processing in ER, ribosome, retinol metabolism and steroid and terpenoid backbone biosynthesis pathways and significant down-regulation of several signaling pathways, such cAMP signaling, notch signaling, cGMP-PKG signaling, apelin signaling pathways were observed between Ctrl HSD-fed vs Ctrl CHOW-fed mice. These pathways are significantly changed in the opposite direction between Pkl KO HSD-fed vs Ctrl HSD-fed mice. These pathways with the reversed gene expression changes indicated the changes in WAT in response to Pkl KO. For instance, the reverse of steroid and terpenoid backbone biosynthesis pathways implicated the potential link between Pkl and whole-body steroid hemostasis. Notably, in both Ctrl HSD-fed vs Ctrl CHOW-fed and Pkl KO HSD-fed vs Ctrl HSD-fed mice, glycolysis, peroxisome, BCAAs degradation, fatty acid degradation and elongation pathways are significantly up-regulated in WAT, while regulation of actin cytoskeleton pathway is significantly down-regulated. The gene expression changes in these pathways are enhanced rather than reversed with Pkl KO, suggesting the complementary response of these pathways to the hepatic gene expression changes. This is exemplified by the enhanced up-regulated glycolysis pathway, as its up-regulation in Ctrl HSD-fed vs Ctrl CHOW-fed mice is due to the excessive amount of glucose intake in the diet. The up-regulation in Pkl KO HSD-fed vs Ctrl HSD-fed mice implicated that the decreased glucose intake in the liver in response to Pkl KO is complemented by the further enhanced glycolysis in WAT.

In the muscle tissue, it was observed that the Pkl KO reversed the down-regulation of metabolic pathways, including glycolysis, galactose metabolism, fructose and mannose metabolism, amino and nucleotide sugar metabolism and fatty acid degradation, and other pathways such as protein processing in ER, antigen processing and presentation, mitophagy, phagosome and spliceosome pathways. In the heart tissue, the Pkl KO reversed the down-regulation of antigen processing and presentation, phagosome, cytokine-cytokine receptor and ECM-receptor interaction pathways and attenuated the expression of the up-regulated pathways, including cardiac muscle contraction, peroxisome, TCA cycle, thermogenesis, oxidative phosphorylation. Notably, no pathway in muscle or heart showed enhanced transcriptomic changes after Pkl KO in response to HSD. In summary, it was concluded that the Pkl KO reversed the transcriptomic changes induced by HSD in extrahepatic tissues, and the affected pathways are mostly related to protein processing, carbohydrate metabolism, fatty acids metabolism, and oxidative phosphorylation as well as some immune and cell recognition associated pathways.

Drug repositioning for inhibition of Pklr in vitro

Based on the *in vitro* analysis in a previous study (Lee et al., 2017; Liu et al., 2019) and the *in vivo* analysis in the present study, it was observed that PKLR is a promising drug target for treatment of NAFLD. Hence, therapeutic agents that inhibit the expression of PKLR are a promising NAFLD treatment strategy. A drug repositioning study was carried out to identify a therapeutic agent that can be tested in clinical trials. In this context, a computational pipeline for repositioning of small molecule drugs that can effectively inhibit Pklr was developed using the data in the Connectivity Map (Subramanian et al., 2017). In brief, human liver tissue RNA-Seq data obtained from 110 subjects in the GTEx database and HepG2 cell line RNA-Seq data where PKLR was inhibited/over-expressed were analysed, and a PKLR-related consensus gene signature was identified. This consensus gene signature was compared to the gene expression profiles of more than 1800 compound-perturbed-HepG2 from the LINCS L1000 database and the potential inhibitors that show similar gene expression profile was identified. As a result, the ten (10) drugs with the most negative expression correlation with the PKLR-related consensus gene signature were selected and these drugs were identified as potential PKLR inhibitors.

All ten drugs were evaluated by performing a literature review and it was found that some of these drugs have been associated with fatty acid metabolism. For instance, Azacitidine, which was reported as a DNA methyltransferase inhibitor, has been approved by the US FDA for the treatment of all subtypes of myelodysplastic syndromes (MDS) (Muller and Florek, 2010). It has also been found that the drug reduces the expression level of a list of genes controlling fatty acid biosynthesis in *vitro* and Pcsk9 (Poirier et al., 2014). In the high-glucose-cultured HepG2 cell model, the agent decreases the expression level of another key DNL gene, Fasn (Hosseini et al., 2020). Among the other 9 compounds, six of them turned out to be kinase inhibitors, indicating their potential effect on PKLR. Additionally, AS-601245 and JNK-IN-5A were reported as the kinase inhibitor of c-Jun N-terminal kinases (JNK) (Cerbone et al., 2012).

To determine the inhibitory effects of these drugs on the expression level of PKLR, HepG2 was selected as the *in vitro* model for coherence. Nine out of the ten drug, i.e. BX-912, PLX-4720, QL-XII-47, Azacitidine, CGK-733, AS-601245, PF-477736, JNK-IN-5A and Dabrafenib, were commercially available and thus purchased. HepG2 cells were treated with these drugs for two days with different dosages due to their potential cellular toxicities (Fig. 5), and the PKLR protein level was the measured by western blotting. The level of protein expression of PKLR of the drug-treated groups was compared to that of the Ctrl group after normalizing with β-actin. As shown in Fig. 2A, six of the nine tested drugs (BX-912, PLX-4720, Azacitidine, CGK-733, AS-601245, JNK-IN-5A) significantly down-regulated the protein expression of PKLR ($P < 0.05$).

To further analyse the effect of these drugs on lipid accumulation via the DNL pathway, steatosis was induced to HepG2 by treating the cells with high glucose medium (DMEM), insulin and T0901317 (Hansmannel et al., 2006). After 7 days of drug treatment, eight of the nine tested drugs (BX-912, PLX-4720, QL-XII-47, Azacitidine, AS-601245, PF-477736, JNK-IN-5A, Dabrafenib) significantly decreased the protein expression level of PKLR ($P < 0.05$; Fig. 2B). Six of the nine tested drugs (BX-912, PLX-4720, Azacitidine, PF-477736, JNK-IN-5A, Dabrafenib) also significantly reduced the protein expression level of FASN ($P < 0.05$; Fig. 2C). Specifically, BX-912 and JNK-IN-5A treated groups showed a dramatic reduction in the protein expression level of both PKLR and FASN in the steatosis model. Therefore, the effects of these two drugs on the *de novo* synthesis of lipids were investigated based on the steatosis-induced HepG2 model. After 7 days of drug treatment, both drugs significantly decreased the content of TGs (normalized by cell viability) in the HepG2 cells. Taken together, it was shown that BX-912 and JNK-IN-5A effectively alleviate the protein expression of PKLR and FASN as well as TG levels in HepG2 cell lines, and thus, it was decided to

perform *in vivo* evaluations to test the effect of these two drugs.

The treatment of rats with the therapeutic agents

To evaluate the potential toxicity of BX-912 and JNK-IN-5A in animals, three groups of 12-week-old rats (n = 5) were raised. The first group was fed with CHOW (Ctrl) for seven days. The second group was fed with CHOW plus BX-912 (30 mg/kg) for seven days. The third group was fed with CHOW plus JNK-IN-5A (30 mg/kg) for seven days. After the seven days, all rats were sacrificed, and tissue samples from major organs and blood samples were collected for biosafety evaluation. The frequencies of micronucleated polychromatic erythrocytes (MNPCEs) in the blood samples were analysed, and it was found that the frequencies of MNPCEs in the drug-treated groups were not statistically different (p>0.05) when compared to Ctrl animals. The analysis indicated that both tested agents exhibited non-genotoxic potential. In addition, histopathological examinations were performed using the tissue samples obtained from the rats and it was found that kidney, small intestine, large intestine, stomach, heart, pancreas, muscle tissues and liver from all three rat groups exhibited normal histological structures. Moreover, immunohistochemical (IHC) examination on the liver tissues from the rats was performed and negative 8-OH-dG expression was observed in all three groups. Therefore, it was concluded that both BX-912 and JNK-IN-5A had no side effects on the rats.

Next, the efficacy of these two drugs in a steatosis rat study was investigated by feeding the animals with HSD. Five groups of 12-week-old rats (n = 5) were raised. For two weeks, the first group was fed with CHOW and the remaining four groups were fed with HSD. At the end of week 2, the CHOW-fed rats (n = 5) and one group of HSD-fed rats (n = 5) were sacrificed for histopathological examination and it was confirmed that the HSD fed rats had already developed hepatic steatosis. One of the remaining groups was fed with HSD (HSD 3w) for seven days. Another one of the remaining groups was fed with HSD plus BX-912 (30 mg/kg) for seven days. The last one of the remaining groups was fed with HSD plus JNK-IN-5A (30 mg/kg) for seven days. After said seven days, all three groups were sacrificed, and tissue samples from the liver and other major organs and blood samples were collected for efficacy examination. A histopathological and immunohistochemical analysis was performed on the liver samples and other major organs obtained from the three rat groups. A standard histological structure on the small intestine, large intestine, stomach, heart and muscle tissues was observed in the examination of the drug-treated groups, and no toxic effect of the drugs on these tissues was found. In the rats from HSD 3w group, severe degeneration was detected in the renal tubule epithelium and severe fattening was detected in the parenchyma cells in the pancreatic tissues. In contrast, only mild degeneration in renal tubule epithelium and mild fattening in the parenchyma cells of pancreas tissues were detected in the drug-treated groups. In addition, severe degeneration and steatosis in hepatocytes in liver tissues of the rats from the HSD 3w group were observed. Mild degeneration and moderate steatosis were observed in hepatocytes in liver tissues of the HSD-fed rats treated with BX-12, whereas both degeneration and steatosis were mild in hepatocytes in liver tissues of the JNK-IN-5A-treated group (Fig. 3). Moreover, strong 8-OH-dG expression was observed in hepatocytes in liver tissues of the rats from HSD 3w group. In the drug-treated groups, the 8-OH-dG expression was however significantly decreased (Fig. 3).

MTT assay

Both BX-912 and JNK-IN-5A significantly reduced the viability of liver cancer cells in the MTT assay (Fig. 4).

## Discussion

In the study presented above, the distinct dietary responses in the liver of HSD-fed and HFD-fed mice compared to CHOW-fed mice were revealed based on transcriptomics data and CNs, and it was shown that HSD-fed mice, rather than HFD-fed mice, exhibit gene expression changes similar to those of mild and moderate NAFLD patients. It was found that glycolysis, insulin related pathways and fatty acid biosynthesis are specifically enhanced in livers of HSD-fed mice together with the elevated hepatic DNL and insulin resistance, which is consistent with the previous knowledge (Shillabeer et al., 1990). The analysis demonstrated that HSD-fed mice is a better model to study NAFLD compared to HFD-fed model, which is in good agreement with the previous studies (Basaranoglu et al., 2015; Mardinoglu et al., 2018).

In the second mice experiment, in which the critical role of Pkl in liver fat development was studied, it was observed that glycolysis, steroid biosynthesis and insulin signaling pathways are enhanced in livers of HSD-fed mice and attenuated liver fat in Pkl KO HSD-fed mice. Enhancement of glycolysis and insulin resistance is a hallmark of NAFLD (Fabbrini et al., 2010), and the inhibition of these two pathways in Pkl KO mice highlighted the potential role of Pklr as a therapeutic target for NAFLD treatment. In fact, a recent study transfected shRNA and plasmid to respectively inhibit and over-express Pklr in mice liver, and demonstrated that Pklr is causal in developing insulin resistance (Chella Krishnan et al., 2021). In addition, the reverse effect of the steroid biosynthesis suggested a potential link between hepatic Pklr and steroid hormone homeostasis, known to be involved in the development of NAFLD (Charni-Natan et al., 2019).

To identify a small molecule drug that can be used to treat NAFLD via inhibition of Pklr, a drug repositioning pipeline was established and candidate drugs

targeting specific genes were found by integrating *in vitro* and *in vivo* gene expression signatures. Ten (10) different small molecule drugs were first selected based on predicted inhibition of Pklr. In an *in vitro* steatosis model, the inhibitory effect of six of them was confirmed. In addition, the two most effective small molecules in *in vitro,* namely BX-912 and JNK-IN-5A, were tested in a rat animal study. Both drugs significantly decreased the hepatic Pklr expression, liver fat and oxidative stress *in vivo* without causing noticeable side effect in the animals. JNK-IN-5A was more effective against steatosis (fatty liver) than BX-912. The results seen in the MTT assay extends the medical indication to hepatocellular carcinoma (HCC).

## REFERENCES

Asher, G., and Schibler, U. (2011). Crosstalk between components of circadian and metabolic cycles in mammals. Cell Metab 13, 125-137.

Basaranoglu, M., Basaranoglu, G., and Bugianesi, E. (2015). Carbohydrate intake and nonalcoholic fatty liver disease: fructose as a weapon of mass destruction. Hepatobiliary Surg Nutr 4, 109-116.

Bray, N.L., Pimentel, H., Melsted, P., and Pachter, L. (2016). Near-optimal probabilistic RNA-seq quantification. Nat Biotechnol 34, 525-527.

Cerbone, A., Toaldo, C., Pizzimenti, S., Pettazzoni, P., Dianzani, C., Minelli, R., Ciamporcero, E., Roma, G., Dianzani, M.U., Canaparo, R., et al. (2012). AS601245, an Anti-Inflammatory JNK Inhibitor, and Clofibrate Have a Synergistic Effect in Inducing Cell Responses and in Affecting the Gene Expression Profile in CaCo-2 Colon Cancer Cells. PPAR Res 2012, 269751.

Charni-Natan, M., Aloni-Grinstein, R., Osher, E., and Rotter, V. (2019). Liver and Steroid Hormones-Can a Touch of p53 Make a Difference? Front Endocrinol (Lausanne) 10, 374.

Chella Krishnan, K., Floyd, R.R., Sabir, S., Jayasekera, D.W., Leon-Mimila, P.V., Jones, A.E., Cortez, A.A., Shravah, V., Peterfy, M., Stiles, L., et al. (2021). Liver Pyruvate Kinase Promotes NAFLD/NASH in Both Mice and Humans in a Sex-Specific Manner. Cell Mol Gastroenterol Hepatol 11, 389-406.

Chella Krishnan, K., Kurt, Z., Barrere-Cain, R., Sabir, S., Das, A., Floyd, R., Vergnes, L., Zhao, Y., Che, N., Charugundla, S., et al. (2018). Integration of Multi-omics Data from Mouse Diversity Panel Highlights Mitochondrial Dysfunction in Non-alcoholic Fatty Liver Disease. Cell Syst 6, 103-115 e107.

Chen, E.Y., Tan, C.M., Kou, Y., Duan, Q., Wang, Z., Meirelles, G.V., Clark, N.R., and Ma'ayan, A. (2013). Enrichr: interactive and collaborative HTML5 gene list enrichment analysis tool. BMC Bioinformatics 14, 128.

Chen, X., and Yang, W. (2015). Branched-chain amino acids and the association with type 2 diabetes. J Diabetes Investig 6, 369-370.

Fabbrini, E., Sullivan, S., and Klein, S. (2010). Obesity and nonalcoholic fatty liver disease: biochemical, metabolic, and clinical implications. Hepatology 51, 679-689.

Gaggini, M., Carli, F., Rosso, C., Buzzigoli, E., Marietti, M., Della Latta, V., Ciociaro, D., Abate, M.L., Gambino, R., Cassader, M., et al. (2018). Altered amino acid concentrations in NAFLD: Impact of obesity and insulin resistance. Hepatology 67, 145-158.

Gluchowski, N.L., Becuwe, M., Walther, T.C., and Farese, R.V., Jr. (2017). Lipid droplets and liver disease: from basic biology to clinical implications. Nat Rev Gastroenterol Hepatol 14, 343-355.

Govaere, O., Cockell, S., Tiniakos, D., Queen, R., Younes, R., Vacca, M., Alexander, L., Ravaioli, F., Palmer, J., Petta, S., et al. (2020). Transcriptomic profiling across the nonalcoholic fatty liver disease spectrum reveals gene signatures for steatohepatitis and fibrosis. Sci Transl Med 12.

Haas, J.T., Francque, S., and Staels, B. (2016). Pathophysiology and Mechanisms of Nonalcoholic Fatty Liver Disease. Annu Rev Physiol 78, 181-205.

Hansmannel, F., Mordier, S., and Iynedjian, P.B. (2006). Insulin induction of glucokinase and fatty acid synthase in hepatocytes: analysis of the roles of sterol-regulatory-element-binding protein-1c and liver X receptor. Biochem J 399, 275-283.

Hosseini, H., Teimouri, M., Shabani, M., Koushki, M., Babaei Khorzoughi, R., Namvarjah, F., Izadi, P., and Meshkani, R. (2020). Resveratrol alleviates nonalcoholic fatty liver disease through epigenetic modification of the Nrf2 signaling pathway. Int J Biochem Cell Biol 119, 105667.

Kanehisa, M., Furumichi, M., Sato, Y., Ishiguro-Watanabe, M., and Tanabe, M. (2021). KEGG: integrating viruses and cellular organisms. Nucleic Acids Res 49, D545-D551.

Koonen, D.P., Jacobs, R.L., Febbraio, M., Young, M.E., Soltys, C.L., Ong, H., Vance, D.E., and Dyck, J.R. (2007). Increased hepatic CD36 expression contributes to dyslipidemia associated with diet-induced obesity. Diabetes 56, 2863-2871.

Kuleshov, M.V., Jones, M.R., Rouillard, A.D., Fernandez, N.F., Duan, Q., Wang, Z., Koplev, S., Jenkins, S.L., Jagodnik, K.M., Lachmann, A., et al. (2016). Enrichr: a comprehensive gene set enrichment analysis web server 2016 update. Nucleic Acids Res 44, W90-97.

Lee, C.H., Cook, S., Lee, J.S., and Han, B. (2016). Comparison of Two Meta-Analysis Methods: Inverse-Variance-Weighted Average and Weighted Sum of Z-Scores. Genomics Inform 14, 173-180.

Lee, S., Zhang, C., Liu, Z., Klevstig, M., Mukhopadhyay, B., Bergentall, M., Cinar, R., Stahlman, M., Sikanic, N., Park, J.K., et al. (2017). Network analyses identify liver-specific targets for treating liver diseases. Mol Syst Biol 13, 938.

Lee, Y.K., Park, J.E., Lee, M., and Hardwick, J.P. (2018). Hepatic lipid homeostasis by peroxisome proliferator-activated receptor gamma 2. Liver Res 2,

209-215.

Liu, Z., Zhang, C., Lee, S., Kim, W., Klevstig, M., Harzandi, A.M., Sikanic, N., Arif, M., Stahlman, M., Nielsen, J., et al. (2019). Pyruvate kinase L/R is a regulator of lipid metabolism and mitochondrial function. Metab Eng 52, 263-272.

Love, M.I., Huber, W., and Anders, S. (2014). Moderated estimation of fold change and dispersion for RNA-seq data with DESeq2. Genome Biol 15, 550.

Mardinoglu, A., Bjornson, E., Zhang, C., Klevstig, M., Soderlund, S., Stahlman, M., Adiels, M., Hakkarainen, A., Lundbom, N., Kilicarslan, M., et al. (2017). Personal model-assisted identification of NAD(+) and glutathione metabolism as intervention target in NAFLD. Mol Syst Biol 13, 916.

Mardinoglu, A., Wu, H., Bjornson, E., Zhang, C., Hakkarainen, A., Rasanen, S.M., Lee, S., Mancina, R.M., Bergentall, M., Pietilainen, K.H., et al. (2018). An Integrated Understanding of the Rapid Metabolic Benefits of a Carbohydrate-Restricted Diet on Hepatic Steatosis in Humans. Cell Metab 27, 559-571 e555.

Muller, A., and Florek, M. (2010). 5-Azacytidine/Azacitidine. Recent Results Cancer Res 184, 159-170.

Poirier, S., Samami, S., Mamarbachi, M., Demers, A., Chang, T.Y., Vance, D.E., Hatch, G.M., and Mayer, G. (2014). The epigenetic drug 5-azacytidine interferes with cholesterol and lipid metabolism. J Biol Chem 289, 18736-18751.

Shillabeer, G., Hornford, J., Forden, J.M., Wong, N.C., and Lau, D.C. (1990). Hepatic and adipose tissue lipogenic enzyme mRNA levels are suppressed by high fat diets in the rat. J Lipid Res 31, 623-631.

Stenvers, D.J., Scheer, F., Schrauwen, P., la Fleur, S.E., and Kalsbeek, A. (2019). Circadian clocks and insulin resistance. Nat Rev Endocrinol 15, 75-89.

Subramanian, A., Narayan, R., Corsello, S.M., Peck, D.D., Natoli, T.E., Lu, X., Gould, J., Davis, J.F., Tubelli, A.A., Asiedu, J.K., et al. (2017). A Next Generation Connectivity Map: L1000 Platform and the First 1,000,000 Profiles. Cell 171, 1437-1452 e1417.

Traag, V.A., Waltman, L., and van Eck, N.J. (2019). From Louvain to Leiden: guaranteeing well-connected communities. Sci Rep 9, 5233.

Udoh, U.S., Valcin, J.A., Swain, T.M., Filiano, A.N., Gamble, K.L., Young, M.E., and Bailey, S.M. (2018). Genetic deletion of the circadian clock transcription factor BMAL1 and chronic alcohol consumption differentially alter hepatic glycogen in mice. Am J Physiol Gastrointest Liver Physiol 314, G431-G447.

Varemo, L., Nielsen, J., and Nookaew, I. (2013). Enriching the gene set analysis of genome-wide data by incorporating directionality of gene expression and combining statistical hypotheses and methods. Nucleic Acids Res 41, 4378-4391.

Virtanen, P., Gommers, R., Oliphant, T.E., Haberland, M., Reddy, T., Cournapeau, D., Burovski, E., Peterson, P., Weckesser, W., Bright, J., et al. (2020). SciPy 1.0: fundamental algorithms for scientific computing in Python. Nat Methods 17, 261-272.

Zerbino, D.R., Achuthan, P., Akanni, W., Amode, M.R., Barrell, D., Bhai, J., Billis, K., Cummins, C., Gall, A., Giron, C.G., et al. (2018). Ensembl 2018. Nucleic Acids Res 46, D754-D761.

Zhang, M., and Zhang, S. (2020). T Cells in Fibrosis and Fibrotic Diseases. Front Immunol 11, 1142.

Zhao, X.K., Yu, L., Cheng, M.L., Che, P., Lu, Y.Y., Zhang, Q., Mu, M., Li, H., Zhu, L.L., Zhu, J.J., et al. (2017). Focal Adhesion Kinase Regulates Hepatic Stellate Cell Activation and Liver Fibrosis. Sci Rep 7, 4032.

## Claims

1. A compound of formula (I)

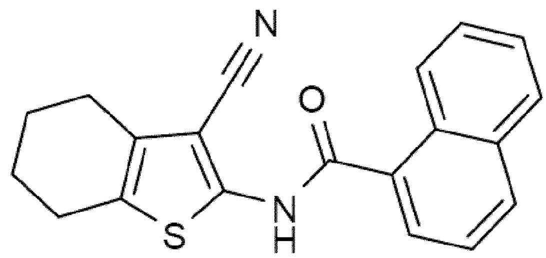

(I)

or a pharmaceutically acceptable salt thereof for use in a method of treatment of fatty liver disease or hepatocellular carcinoma (HCC).

2. The compound for use according to claim 1, wherein the method of treatment comprises oral administration of the compound.

3. The compound for use according to claim 1 or 2, wherein said fatty liver disease is non-alcoholic fatty liver disease (NAFLD).

4. The compound for use according to claim 3, wherein said NAFLD has progressed to non-alcoholic steatohepatitis (NASH).

## Patentansprüche

1. Verbindung mit der Formel (I)

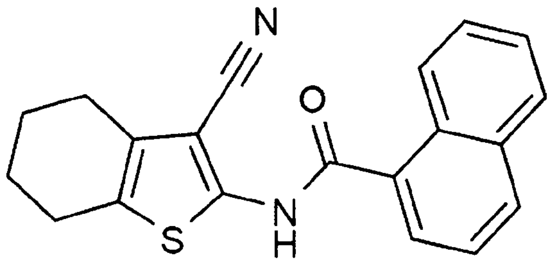

(I)

oder ein pharmazeutisch akzeptables Salz davon zur Verwendung in einem Verfahren zur Behandlung einer Fettlebererkrankung oder eines Leberzellkarzinoms (hepatozelluläres Karzinom, HZK).

2. Verbindung zur Verwendung nach Anspruch 1, wobei das Verfahren zur Behandlung eine orale Verabreichung der Verbindung umfasst.

3. Verbindung zur Verwendung nach Anspruch 1 oder 2, wobei es sich bei der Fettlebererkrankung um eine nichtalkoholische Fettlebererkrankung (NAFLD) handelt.

4. Verbindung zur Verwendung nach Anspruch 3, wobei die NAFLD zu einer nichtalkoholischen Steatohepatitis (NASH) fortgeschritten ist.

## Revendications

1. Composé de formule (I)

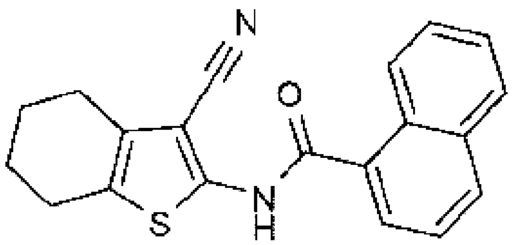

(I)

ou un de ses sels pharmaceutiquement acceptables destiné à être utilisé dans un procédé de traitement de maladie du foie gras ou du carcinome hépatocellulaire (HCC).

2. Composé destiné à être utilisé selon la revendication 1, dans lequel le procédé de traitement comprend l'administration orale du composé.

3. Composé destiné à être utilisé selon la revendication 1 ou 2, dans lequel ladite maladie du foie gras est la maladie du foie gras non alcoolique (NAFLD).

4. Composé destiné à être utilisé selon la revendication 3, dans lequel ladite NAFLD a progressé en une stéatohépatite non alcoolique (NASH).

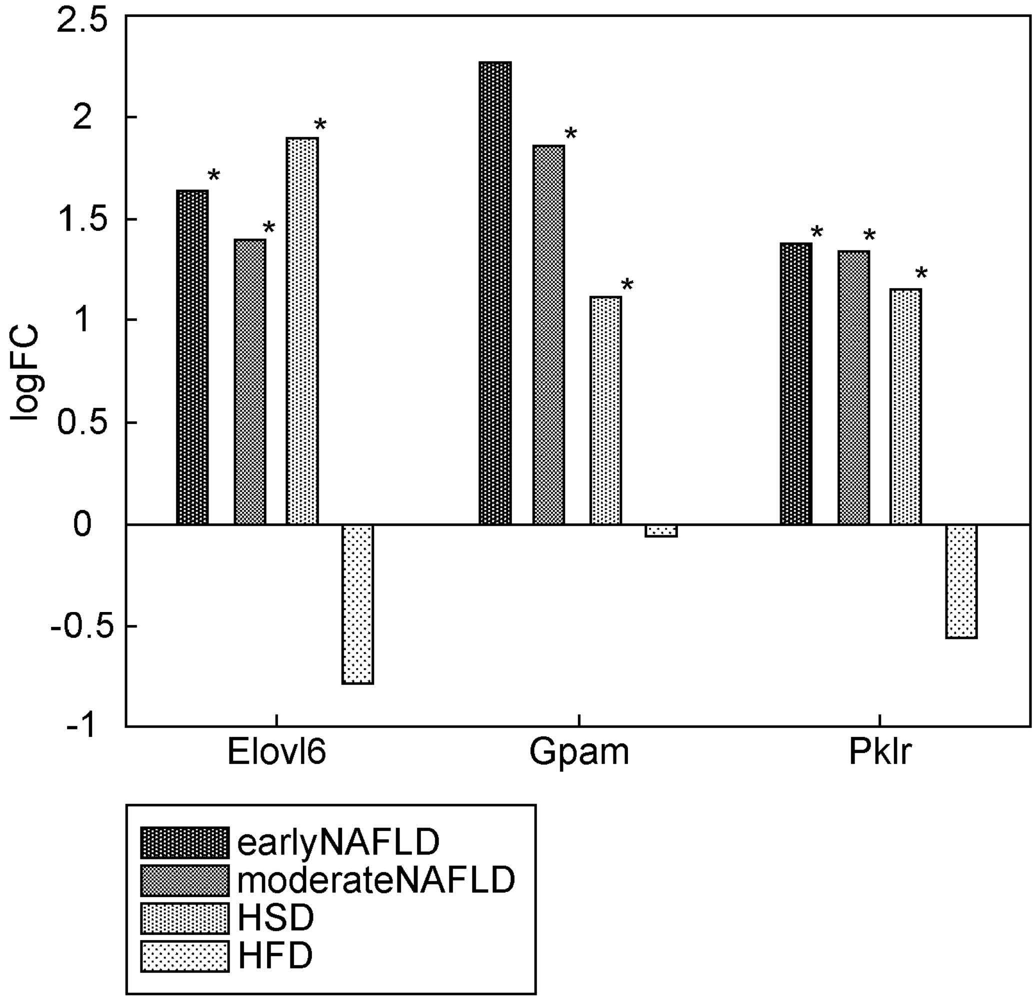
2.5
2
1.5
1
0.5
0
-0.5
-1
logFC
*
*
*
*
*
*
*
Elovl6
Gpam
Pklr
earlyNAFLD
moderateNAFLD
HSD
HFD

Fig. 1

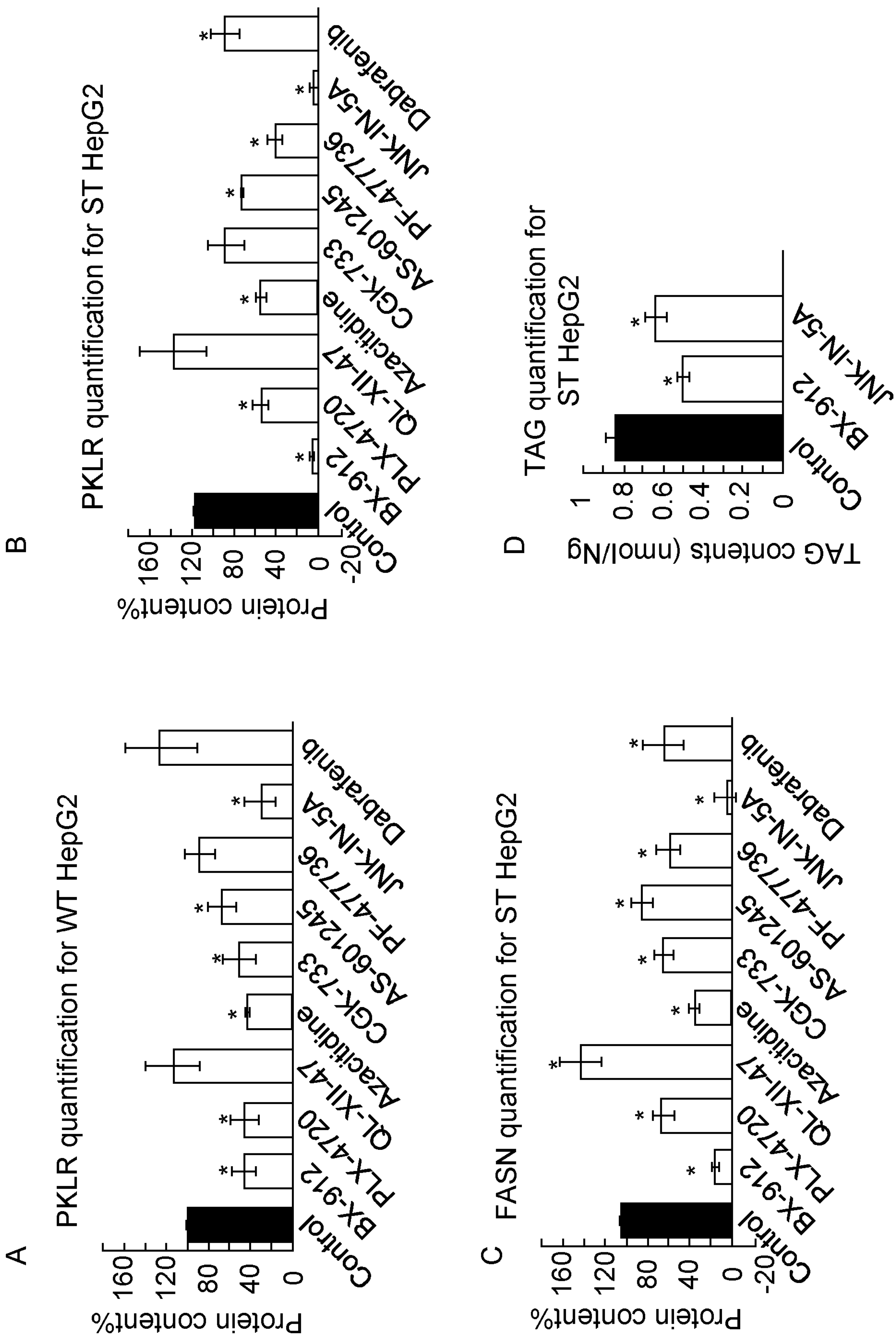
A
PKLR quantification for WT HepG2
Protein content%
160
120
80
40
0
Control
BX-912
PLX-4720
QL-XII-47
Azacitidine
CGK-733
AS-601245
PF-477736
JNK-IN-5A
Dabrafenib
B
PKLR quantification for ST HepG2
Protein content%
160
120
80
40
0
-20
C
FASN quantification for ST HepG2
Protein content%
160
120
80
40
0
-20
D
TAG quantification for
ST HepG2
TAG contents (nmol/Ng
1
0.8
0.6
0.4
0.2
0
Control
BX-912
JNK-IN-5A

Fig. 2

| | Degeneration | Lipidosis | 8-OH-dG expression |
|---|---|---|---|
| HSD | +++ | +++ | +++ |
| HSD plus BX-912 | + | ++ | + |
| HSD plus JNK-IN-5A | + | + | + |

Fig. 3

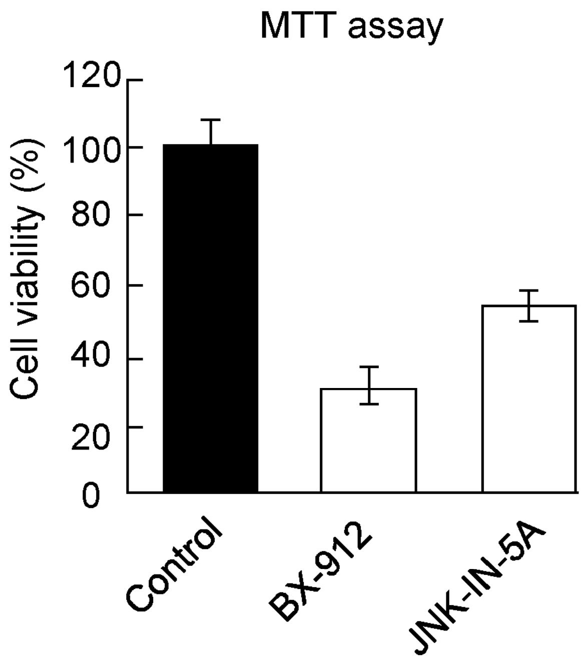


Fig. 4

| Drug name | Two-day treatment WB (μM) | One-week treatment WB (μM) | One-week treatment TAG (μM) |
|---|---|---|---|
| BX-912 | 10 | 10 | 5 |
| PLX-4720 | 2.5 | 2.5 | / |
| QL-XII-47 | 5 | 5 | / |
| Azacitidine | 5 | 5 | / |
| CGK-733 | 5 | 2.5 | / |
| AS-601245 | 5 | 5 | / |
| PF-477736 | 2.5 | 2.5 | / |
| JNK-IN-5A | 5 | 5 | 5 |
| Dabrafenib | 5 | 5 | / |

Fig. 5

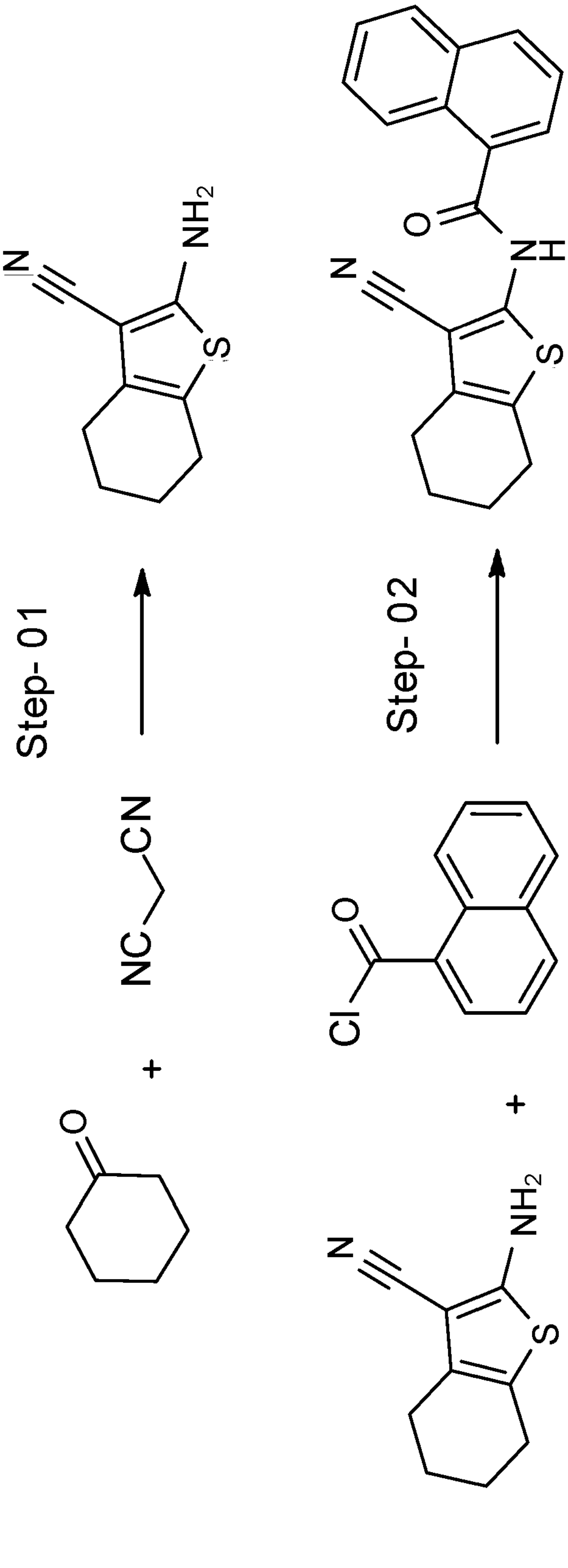


Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019034657 A

### Non-patent literature cited in the description


- **ASHER, G. ; SCHIBLER, U.** Crosstalk between components of circadian and metabolic cycles in mammals. *Cell Metab*, 2011, vol. 13, 125-137
- **BASARANOGLU, M. ; BASARANOGLU, G. ; BUGIANESI, E.** Carbohydrate intake and nonalcoholic fatty liver disease: fructose as a weapon of mass destruction. *Hepatobiliary Surg Nutr*, 2015, vol. 4, 109-116
- **BRAY, N.L. ; PIMENTEL, H. ; MELSTED, P. ; PACHTER, L.** Near-optimal probabilistic RNA-seq quantification. *Nat Biotechnol*, 2016, vol. 34, 525-527
- **CERBONE, A. ; TOALDO, C. ; PIZZIMENTI, S. ; PETTAZZONI, P. ; DIANZANI, C. ; MINELLI, R. ; CIAMPORCERO, E. ; ROMA, G. ; DIANZANI, M.U. ; CANAPARO, R. et al.** AS601245, an Anti-Inflammatory JNK Inhibitor, and Clofibrate Have a Synergistic Effect in Inducing Cell Responses and in Affecting the Gene Expression Profile in CaCo-2 Colon Cancer Cells. *PPAR Res 2012*, 2012, 269751
- **CHARNI-NATAN, M. ; ALONI-GRINSTEIN, R. ; OSHER, E. ; ROTTER, V.** Liver and Steroid Hormones-Can a Touch of p53 Make a Difference?. *Front Endocrinol (Lausanne)*, 2019, vol. 10, 374
- **CHELLA KRISHNAN, K. ; FLOYD, R.R. ; SABIR, S. ; JAYASEKERA, D.W. ; LEON-MIMILA, P.V. ; JONES, A.E. ; CORTEZ, A.A. ; SHRAVAH, V. ; PETERFY, M. ; STILES, L. et al.** Liver Pyruvate Kinase Promotes NAFLD/NASH in Both Mice and Humans in a Sex-Specific Manner. *Cell Mol Gastroenterol Hepatol*, 2021, vol. 11, 389-406
- **CHELLA KRISHNAN, K. ; KURT, Z. ; BARRERE-CAIN, R. ; SABIR, S. ; DAS, A. ; FLOYD, R. ; VERGNES, L. ; ZHAO, Y. ; CHE, N. ; CHARUGUNDLA, S. et al.** Integration of Multi-omics Data from Mouse Diversity Panel Highlights Mitochondrial Dysfunction in Non-alcoholic Fatty Liver Disease. *Cell Syst*, 2018, vol. 6, 103-115
- **CHEN, E.Y. ; TAN, C.M. ; KOU, Y. ; DUAN, Q. ; WANG, Z. ; MEIRELLES, G.V. ; CLARK, N.R. ; MA'AYAN, A.** Enrichr: interactive and collaborative HTML5 gene list enrichment analysis tool. *BMC Bioinformatics*, 2013, vol. 14, 128
- **CHEN, X. ; YANG, W.** Branched-chain amino acids and the association with type 2 diabetes. *J Diabetes Investig*, 2015, vol. 6, 369-370
- **FABBRINI, E. ; SULLIVAN, S. ; KLEIN, S.** Obesity and nonalcoholic fatty liver disease: biochemical, metabolic, and clinical implications. *Hepatology*, 2010, vol. 51, 679-689
- **GAGGINI, M. ; CARLI, F. ; ROSSO, C. ; BUZZIGOLI, E. ; MARIETTI, M. ; DELLA LATTA, V. ; CIOCIARO, D. ; ABATE, M.L. ; GAMBINO, R. ; CASSADER, M. et al.** Altered amino acid concentrations in NAFLD: Impact of obesity and insulin resistance. *Hepatology*, 2018, vol. 67, 145-158
- **GLUCHOWSKI, N.L. ; BECUWE, M. ; WALTHER, T.C. ; FARESE, R.V., JR.** Lipid droplets and liver disease: from basic biology to clinical implications. *Nat Rev Gastroenterol Hepatol*, 2017, vol. 14, 343-355
- **GOVAERE, O. ; COCKELL, S. ; TINIAKOS, D. ; QUEEN, R. ; YOUNES, R. ; VACCA, M. ; ALEXANDER, L. ; RAVAIOLI, F. ; PALMER, J. ; PETTA, S. et al.** Transcriptomic profiling across the nonalcoholic fatty liver disease spectrum reveals gene signatures for steatohepatitis and fibrosis. *Sci Transl Med*, 2020, vol. 12
- **HAAS, J.T. ; FRANCQUE, S. ; STAELS, B.** Pathophysiology and Mechanisms of Nonalcoholic Fatty Liver Disease. *Annu Rev Physiol*, 2016, vol. 78, 181-205
- **HANSMANNEL, F. ; MORDIER, S. ; IYNEDJIAN, P.B.** Insulin induction of glucokinase and fatty acid synthase in hepatocytes: analysis of the roles of sterol-regulatory-element-binding protein-1c and liver X receptor. *Biochem J*, 2006, vol. 399, 275-283
- **HOSSEINI, H. ; TEIMOURI, M. ; SHABANI, M. ; KOUSHKI, M. ; BABAEI KHORZOUGHI, R. ; NAMVARJAH, F. ; IZADI, P. ; MESHKANI, R.** Resveratrol alleviates non-alcoholic fatty liver disease through epigenetic modification of the Nrf2 signaling pathway. *Int J Biochem Cell Biol*, 2020, vol. 119, 105667

- **KANEHISA, M.** ; **FURUMICHI, M.** ; **SATO, Y.** ; **ISHIGURO-WATANABE, M.** ; **TANABE, M.** KEGG: integrating viruses and cellular organisms. *Nucleic Acids Res*, 2021, vol. 49, D545-D551
- **KOONEN, D.P.** ; **JACOBS, R.L.** ; **FEBBRAIO, M.** ; **YOUNG, M.E.** ; **SOLTYS, C.L.** ; **ONG, H.** ; **VANCE, D.E.** ; **DYCK, J.R.** Increased hepatic CD36 expression contributes to dyslipidemia associated with diet-induced obesity. *Diabetes*, 2007, vol. 56, 2863-2871
- **KULESHOV, M.V.** ; **JONES, M.R.** ; **ROUILLARD, A.D.** ; **FERNANDEZ, N.F.** ; **DUAN, Q.** ; **WANG, Z.** ; **KOPLEV, S.** ; **JENKINS, S.L.** ; **JAGODNIK, K.M.** ; **LACHMANN, A. et al.** Enrichr: a comprehensive gene set enrichment analysis web server 2016 update. *Nucleic Acids Res*, 2016, vol. 44, 90-97
- **LEE, C.H.** ; **COOK, S.** ; **LEE, J.S.** ; **HAN, B.** Comparison of Two Meta-Analysis Methods: Inverse-Variance-Weighted Average and Weighted Sum of Z-Scores. *Genomics Inform*, 2016, vol. 14, 173-180
- **LEE, S.** ; **ZHANG, C.** ; **LIU, Z.** ; **KLEVSTIG, M.** ; **MUKHOPADHYAY, B.** ; **BERGENTALL, M.** ; **CINAR, R.** ; **STAHLMAN, M.** ; **SIKANIC, N.** ; **PARK, J.K. et al.** Network analyses identify liver-specific targets for treating liver diseases. *Mol Syst Biol*, 2017, vol. 13, 938
- **LEE, Y.K.** ; **PARK, J.E.** ; **LEE, M.** ; **HARDWICK, J.P.** Hepatic lipid homeostasis by peroxisome proliferator-activated receptor gamma 2. *Liver Res*, 2018, vol. 2, 209-215
- **LIU, Z.** ; **ZHANG, C.** ; **LEE, S.** ; **KIM, W.** ; **KLEVSTIG, M.** ; **HARZANDI, A.M.** ; **SIKANIC, N.** ; **ARIF, M.** ; **STAHLMAN, M.** ; **NIELSEN, J. et al.** Pyruvate kinase L/R is a regulator of lipid metabolism and mitochondrial function. *Metab Eng*, 2019, vol. 52, 263-272
- **LOVE, M.I.** ; **HUBER, W.** ; **ANDERS, S.** Moderated estimation of fold change and dispersion for RNA-seq data with DESeq2. *Genome Biol*, 2014, vol. 15, 550
- **MARDINOGLU, A.** ; **BJORNSON, E.** ; **ZHANG, C.** ; **KLEVSTIG, M.** ; **SODERLUND, S.** ; **STAHLMAN, M.** ; **ADIELS, M.** ; **HAKKARAINEN, A.** ; **LUNDBOM, N.** ; **KILICARSLAN, M. et al.** Personal model-assisted identification of NAD(+) and glutathione metabolism as intervention target in NAFLD. *Mol Syst Biol*, 2017, vol. 13, 916
- **MARDINOGLU, A.** ; **WU, H.** ; **BJORNSON, E.** ; **ZHANG, C.** ; **HAKKARAINEN, A.** ; **RASANEN, S.M.** ; **LEE, S.** ; **MANCINA, R.M.** ; **BERGENTALL, M.** ; **PIETILAINEN, K.H. et al.** An Integrated Understanding of the Rapid Metabolic Benefits of a Carbohydrate-Restricted Diet on Hepatic Steatosis in Humans. *Cell Metab*, 2018, vol. 27, 559-571
- **MULLER, A.** ; **FLOREK, M.** 5-Azacytidine/Azacitidine. *Recent Results Cancer Res*, 2010, vol. 184, 159-170
- **POIRIER, S.** ; **SAMAMI, S.** ; **MAMARBACHI, M.** ; **DEMERS, A.** ; **CHANG, T.Y.** ; **VANCE, D.E.** ; **HATCH, G.M.** ; **MAYER, G.** The epigenetic drug 5-azacytidine interferes with cholesterol and lipid metabolism. *J Biol Chem*, 2014, vol. 289, 18736-18751
- **SHILLABEER, G.** ; **HORNFORD, J.** ; **FORDEN, J.M.** ; **WONG, N.C.** ; **LAU, D.C.** Hepatic and adipose tissue lipogenic enzyme mRNA levels are suppressed by high fat diets in the rat. *J Lipid Res*, 1990, vol. 31, 623-631
- **STENVERS, D.J.** ; **SCHEER, F.** ; **SCHRAUWEN, P.** ; **LA FLEUR, S.E.** ; **KALSBEEK, A.** Circadian clocks and insulin resistance. *Nat Rev Endocrinol*, 2019, vol. 15, 75-89
- **SUBRAMANIAN, A.** ; **NARAYAN, R.** ; **CORSELLO, S.M.** ; **PECK, D.D.** ; **NATOLI, T.E.** ; **LU, X.** ; **GOULD, J.** ; **DAVIS, J.F.** ; **TUBELLI, A.A.** ; **ASIEDU, J.K. et al.** A Next Generation Connectivity Map: L1000 Platform and the First 1,000,000 Profiles. *Cell*, 2017, vol. 171, 1437-1452
- **TRAAG, V.A.** ; **WALTMAN, L.** ; **VAN ECK, N.J.** From Louvain to Leiden: guaranteeing well-connected communities. *Sci Rep*, 2019, 5233
- **UDOH, U.S.** ; **VALCIN, J.A.** ; **SWAIN, T.M.** ; **FILIANO, A.N.** ; **GAMBLE, K.L.** ; **YOUNG, M.E.** ; **BAILEY, S.M.** Genetic deletion of the circadian clock transcription factor BMAL1 and chronic alcohol consumption differentially alter hepatic glycogen in mice. *Am J Physiol Gastrointest Liver Physiol*, 2018, vol. 314, G431-G447
- **VAREMO, L.** ; **NIELSEN, J.** ; **NOOKAEW, I.** Enriching the gene set analysis of genome-wide data by incorporating directionality of gene expression and combining statistical hypotheses and methods. *Nucleic Acids Res*, 2013, vol. 41, 4378-4391
- **VIRTANEN, P.** ; **GOMMERS, R.** ; **OLIPHANT, T.E.** ; **HABERLAND, M.** ; **REDDY, T.** ; **COURNAPEAU, D.** ; **BUROVSKI, E.** ; **PETERSON, P.** ; **WECKESSER, W.** ; **BRIGHT, J. et al.** SciPy 1.0: fundamental algorithms for scientific computing in Python. *Nat Methods*, 2020, vol. 17, 261-272
- **ZERBINO, D.R.** ; **ACHUTHAN, P.** ; **AKANNI, W.** ; **AMODE, M.R.** ; **BARRELL, D.** ; **BHAI, J.** ; **BILLIS, K.** ; **CUMMINS, C.** ; **GALL, A.** ; **GIRON, C.G. et al.** *Ensembl 2018. Nucleic Acids Res*, 2018, vol. 46, D754-D761
- **ZHANG, M.** ; **ZHANG, S.** T Cells in Fibrosis and Fibrotic Diseases. *Front Immunol*, 2020, vol. 11, 1142
- **ZHAO, X.K.** ; **YU, L.** ; **CHENG, M.L.** ; **CHE, P.** ; **LU, Y.Y.** ; **ZHANG, Q.** ; **MU, M.** ; **LI, H.** ; **ZHU, L.L.** ; **ZHU, J.J. et al.** Focal Adhesion Kinase Regulates Hepatic Stellate Cell Activation and Liver Fibrosis. *Sci Rep*, 2017, vol. 7, 4032